## Europäisches Patentamt
## European Patent Office
## Office européen des brevets

(11) Publication number: **0 137 072**
**B1**

# (12) EUROPEAN PATENT SPECIFICATION

(45) Date of publication of patent specification: **20.05.87**

(51) Int. Cl.⁴: **A 47 C 27/15, A 47 C 27/00**

(21) Application number: **83110180.3**

(22) Date of filing: **12.10.83**

(54) A sleeping mattress.

(43) Date of publication of application:
**17.04.85 Bulletin 85/16**

(45) Publication of the grant of the patent:
**20.05.87 Bulletin 87/21**

(84) Designated Contracting States:
**AT BE CH DE FR GB IT LI LU NL SE**

(56) References cited:
**CH-A- 439 627**
**DE-A-2 240 798**
**FR-A-1 247 975**
**GB-A- 560 508**
**US-A-4 143 435**
**US-A-4 330 892**

(73) Proprietor: **JAPAN LIFE COMPANY LIMITED**
**1-1, 3 chome Higashiikebukuro**
**Toshima-ku Tokyo (JP)**
(73) Proprietor: **ASAHI INDUSTRY COMPANY LIMITED**
**2-775, Ohnumacho**
**Kodairashi Tokyo (JP)**

(72) Inventor: **Yagi, Toshizo**
**No. 271-9, Misonocho**
**Kodairashi Tokyo (JP)**

(74) Representative: **Klingseisen, Franz, Dipl.-Ing. et al**
**Dr. F. Zumstein sen. Dr. E. Assmann Dr. F. Zumstein jun. Dipl.-Ing. F. Klingseisen**
**Bräuhausstrasse 4**
**D-8000 München 2 (DE)**

Courier Press, Leamington Spa, England.

## Description

The invention relates to a sleeping mattress according to the preamble of claim 1.

Such a sleeping mattress is known from US—A—4 143 435. The mattress is made of foam rubber which is provided with a number of round protrusions on its upper and lower surfaces as well as a number of magnets which are disposed in the valleys formed by those protrusions. In the mattress of a foam rubber a corrugated board is disposed therein. Further US—A—4 330 892 shows a mattress which comprises fillings, a plurality of permanent magnet pieces provided on a mounting means, and a covering cloth for covering the above-mentioned components. The magnet pieces generate lines of magnetic force.

It is the object of the invention to improve a sleeping mattress of the above-mentioned kind in such a way that the back bone is kept straight during sleeping and the blood is smoothly circulated due to its magnetic effect to remove stiffness in the shoulders. Further the mattress should be excellent in air permeability and well conditioned in the magnetic curing effect. The mattress should be moderate in hardness balancing as a whole and hold its shape.

This object is achieved by the features in the characterizing part of claim 1.

Embodiments of the invention are mentioned in the subclaims.

An example of the invention is described in more detail in connection with the drawing.

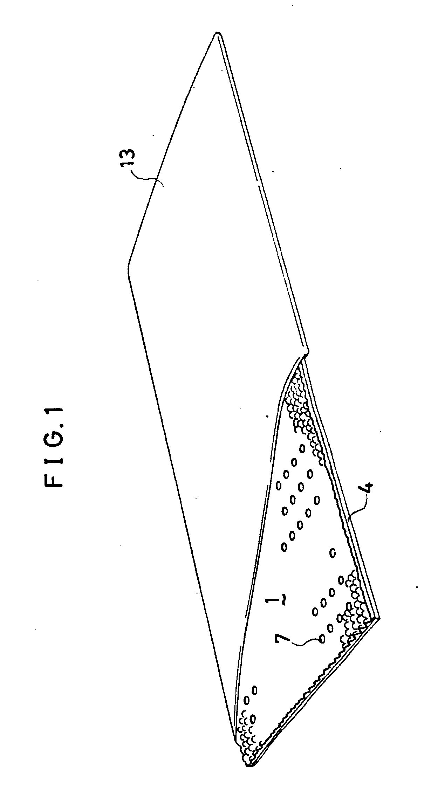

Fig. 1 is a perspective view of the mattress,

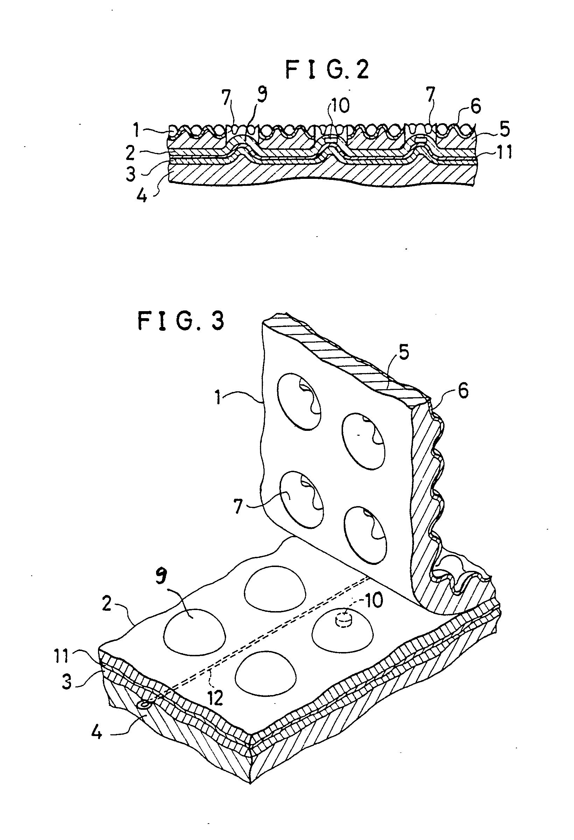

Fig. 2 is a vertical cross section showing the inner structure of the same,

Fig. 3 is a view showing an example of arranging magnets and reinforcing materials,

Fig. 4 is a view showing an example of arranging magnets and steel materials,

Fig. 5A is a view showing magnetic line of force of a conventional magnet, and

Fig. 5B is a view showing magnetic line of force of the invention.

The mattress comprises a structure of four layers in which a first layer 1 is a wave layer, a second layer 2 is a magnetic layer including magnets, a third layer 3 is a compression palm lock layer, and a fourth layer 4 is a cushion layer.

The first layer 1 is for balancing the body weight in uniform dispersion and giving soft massaging effect to the body. This layer comprises a cushion material of about 2 cm in thickness, preferably a urethane form 5 is formed thereon with a thin outer cover 6 and is perforated with holes regularly lengthwise and crosswise, in which the urethane form 5 is provided with a plurality of convexes of wave-like cross-section regularly lengthwise and crosswise, and the thin outer cover 6 is impregnated with resin liquid such as urethane form. Concerning the number of the holes, about 21 × 12 are required for the size of the product being 1950 (length) mm × 930 (width) mm, and about 21 × 20 are required for the size of 1950 × 1300. Permanent magnets as later mentioned are exposed at the holes 7.

The second layer 2 is for naturally straightening the spine while giving the magnetic effect to the body. In this layer, the thin sheet 8 made of urethane form is arranged thereon with hemispherical projections 9 in parallel, and desired ones of the projections 9 are laid with ferrite permanent magnets 10. The second layer 2 is, as later mentioned, made integral with a third layer 3 via hard grains 11 of urethane resin and adhesive so that it holds the shape of the mattress.

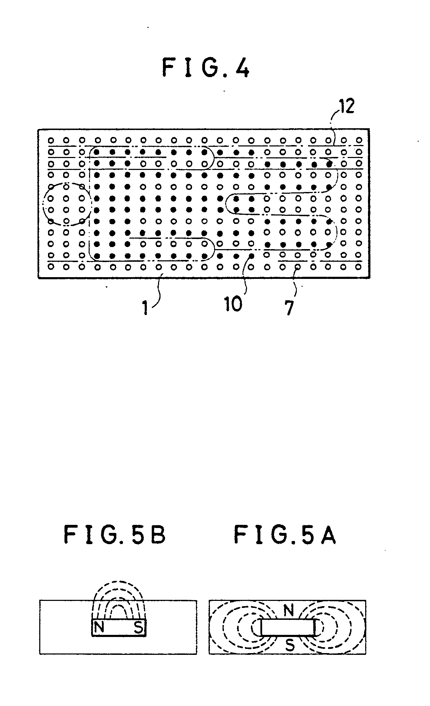

For a permanent magnet 10, used is one which is two poles at one side (magnetism on one side). Thereby, magnetic line of force reaches far (see Fig. 5B) in comparison with the conventional one (see Fig. 5A). Thus, the magnetic effect serves at deeper parts of the human body so that the blood runs actively and the stiffness is removed from the body. In order to provide the magnetic curing effect to the maximum, it is preferable to deposit the permanent magnets 10 having 850 gauss at parts nearest to the human body, that is, at the projections 9. However, the magnets 10 are covered with thin sponge or cloth for preventing unpleasant feeling caused by directly touching the skin. The magnets 10 are not necessarily all arranged within the projections 9, but gathered at the parts corresponding to the shoulders, back, waiste or legs. Fig. 4 illustrates an example of such an arrangement with black marks.

The third layer 3 is for maintaining moderate hardness over the entire mattress in order to prevent the spine from curving due to unnatural sinking of the body. This layer comprises compressed fibers such as palm or the like, and is arranged with reinforcing material, for example, steel wires 12 of around 3 mm in diameter thereover with appropriate space for providing stronger rigidity. For a three-fold type, the steel lines 12 are arranged in division lengthwise. For a non-folding type, the steel wire 12 may be divided appropriately. Further, plastic plate may be used as the reinforcing material, instead of the steel wire 12. The third layer 3 integrally holds grains 11 of hard urethane resin and binding agent between the second layer 2 and itself and forms many concaves and convexes, and the concaves fit within the projections 9, which concaves have the nature of holding shape. In such a manner, the semi-circular shape of the projections 9 is firmly kept and prevented from depression.

The fourth layer 4 is for supporting as a whole the mattress, effecting moderate cushion and softening vibration of the body. This layer is in general made of urethane form.

The mattress composed of the four layers may be a three-fold or non-folding type. In use, the mattress is protected with a cover 13.

Effects brought about by the four layered structure follows.

1) By projections of wave-like cross-section of the first layer and projections 9 of the second layer, the body weight is dispersed in balance while soft massaging effect is obtained. Through the projections and the compressed palm layer of the third layer, evaporation of the moisture by such as sweat and others is accelerated, so that ideal feeling may be effected as being warm in the winter and cool in the summer.

2) The magnets arranged in the second layer act on the human body as follows.

A. Effecting magnetism to the human body
B. Inviting new electricity
C. Generating electric current in the blood
D. Increasing ion in the blood
E. Changing action of autonomic nerve
F. Smoothening circulation of the blood
G. Improving stiffness or ill conditions

3) Grains of hard urethane resin between the second and third layers, and the third layer including the reinforcing material give moderate rigidity over the mattress, and the back bone is kept in straight during lying on the mattress.

4) The lowest urethane form layer gives comfortable cushion feeling.

**Claims**

1. A sleeping mattress comprising a layer (1) made of cushion material formed with projections of wave-like cross-section and permanent magnets (10) distributed over the mattress, characterized in that the first layer (1) is followed by a second layer (2), having hemispherical projections (9) in which the permanent magnets (10) are provided, a third layer (3) stuffed with palm fibers and reinforcing materials, and a fourth layer (4) composed of cushion materials.

2. A sleeping mattress as claimed in Claim 1, wherein a permanent magnet has two poles on one side thereof.

3. A sleeping mattress as claimed in Claim 1, wherein permanent magnets (10) are disposed at parts corresponding to the shoulders, arms, spine, waiste and legs.

4. A sleeping mattress as claimed in Claim 1, wherein a permanent magnet has 850 gauss.

5. A sleeping mattress as claimed in Claim 1, wherein the permanent magnets (10) are positioned within the projections (9).

6. A sleeping mattress as claimed in Claim 1, wherein reinforcing materials are steel wires (12).

7. A sleeping mattress as claimed in Claim 1, wherein the reinforcing materials are plastic plates.

8. A sleeping mattress as claimed in Claim 1, wherein the hemispherical shape of the projections (9) holding the magnets (10) therein is held by means of convexes formed in a compressed palm lock layer.

**Patentansprüche**

1. Matratze mit einer Schicht (1) aus einem Polstermaterial, die mit Vorsprüngen vo wellenartigem Querschnitt versehen ist, und mit Permanentmagneten (10), die über die Matratze verteilt sind, dadurch gekennzeichnet, daß auf die erste Schicht (1) eine zweite Schicht (2), die halbkugelförmige Vorsprünge (9) aufweist, in denen die Permanentmagneten (10) vorgesehen sind, eine dritte Schicht (3), die mit Palmfasern und Verstärkungsmaterialien ausgefüllt ist, und eine vierte Schicht (4) folgen, die aus Polstermaterialien besteht.

2. Matratze nach Anspruch 1, bei der ein Permanentmagnet zwei Pole auf seiner einen Seite aufweist.

3. Matratze nach Anspruch 1, bei der die Permanentmagneten (10) an Teilen angeordnet sind, die den Schultern, Armen, der Wirbelsäule, der Taille und den Beinen entsprechen.

4. Matratze nach Anspruch 1, bei der ein Permanentmagnet 850 Gauss hat.

5. Matratze nach Anspruch 1, bei der die Permanentmagneten (10) in den Vorsprüngen (9) angeordnet sind.

6. Matratze nach Anspruch 1, bei der die Verstärkungsmaterialien Stahldrähte (12) sind.

7. Matratze nach Anspruch 1, bei der die Verstärkungsmaterialien Kunststoffplatten sind.

8. Matratze nach Anspruch 1, bei der die Halbkugelform der Vorsprünge (9), die die Magneten (10) darin halten, über konvexe Aussparungen beibehalten wird, die in einer verdichteten Palmwolleschicht ausgebildet sind.

**Revendications**

1. Matelas comprenant une couche (1) constituée par un matériau de rembourrage présentant des saillies de section ondulée, ainsi que des aimants permanents (10) répartis sur le matelas, caractérisé par le fait que la première couche (1) est suivie d'une deuxième couche (2) comportant des protubérances hémisphériques (9) dans les-quelles les aimants permanents (10) sont logés, d'une troisième couche (3) rembourrée par des fibres de palme et des matériaux de renfort, et d'une quatrième couche (4) constituée par des matériaux de rembourrage.

2. Matelas selon la revendication 1, dans lequel un aimant permanent présente deux pôles sur l'un des ses côtés.

3. Matelas selon la revendication 1, dans lequel des aimants permanents (10) sont disposés en des endroits correspondant aux épaules, aux bras, à l'épine dorsale, à la taille et aux jambes.

4. Matelas selon la revendication 1, dans lequel un aimant permanent a un champ magnétique de 850 gauss.

5. Matelas selon la revendication 1, dans lequel les aimants permanents (10) sont incorporés dans les protubérances (9).

6. Matelas selon la revendication 1, dans lequel des matériaux de renfort sont des fils d'acier (12).

7. Matelas selon la revendication 1, dans lequel les matériaux de renfort sont des panneaux de matière plastique.

8. Matelas selon la revendication 1, dans lequel la forme hémisphérique des protubérances (9) renfermant les aimants (10) est maintenue au moyen de configurations convexes formées dans une couche de blocage en palme comprimée.

FIG.1

13

7

1

4

0 137 072

0 137 072

FIG. 2

FIG. 3

2

# FIG. 4

# FIG. 5B    FIG. 5A

3